# EUROPÄISCHE PATENTANMELDUNG

(11) **EP 1 486 164 A1**
(43) Veröffentlichungstag der Anmeldung: **15.12.2004**
(21) Anmeldenummer: 04013419.9
(22) Anmeldetag: 08.06.2004
(51) Int. Cl.: A61B 5/0428, A61B 5/0408

(54) **Elektroden-Sensorsystem, insbesondere zur Aufzeichnung eines Elektrokardiogramms**

(30) Priorität: 12.06.2003 DE 10326402
(71) Anmelder: ANTEA Medizintechnik und Informationstechnik GmbH, 76133 Karlsruhe (DE)
(72) Erfinder: Franz, Thilo, 76137 Karlsruhe (DE); Kolb, Gunter, Dipl.-Phys., 76703 Kraichtal (DE)
(74) Vertreter: Lasch, Hartmut Dipl.-Ing.

(57) **Zusammenfassung**

Die Erfindung betrifft ein Elektroden-Sensorsystem zur Messung von elektrischen Potentialen an der Hautoberfläche von Menschen oder Tieren, insbesondere zur Aufzeichnung eines Elektrokardiogramms, mit mehreren, wenigstens eine Elektrode aufweisenden, an der Hautoberfläche anbringbaren Elektroden-Einheiten, und mit einer am Körper lösbar befestigbaren Verteiler-Einheit mit einer Mehrzahl von Anschlußstellen. Letztere sind mit den Elektroden der Elektroden-Einheiten über Kabel verbindbar. Die zu den Anschlußstellen der Verteiler-Einheit führenden Kabel sind in einer Verteiler-Einrichtung zusammengeführt, welche mit einer Meßeinrichtung derart verbindbar ist, daß sämtliche Anschlußstellen der Verteiler-Einheit mit der Meßeinrichtung kommunizieren. Erfindungsgemäß ist vorgesehen, daß die Verteiler-Einheit in Form eines Gürtels ausgebildet ist, welcher um den Körper des Menschen oder Tieres anordbar ist, und daß die Anschlußstellen in Erstreckungsrichtung des Gürtels hintereinander und mit Abstand voneinander angeordnet sind. Die Anschlußstellen des Gürtels sind ferner von Mehrpol-Buchsen bzw. Mehrpol-Streckern gebildet, an welchen die Elektroden der mit mehreren Elektroden ausgestatteten Elektroden-Einheiten über hierzu komplementären Mehrpol-Stecker bzw. Mehrpol-Buchsen jeweils gemeinsam anschließbar sind.

## Beschreibung

Die Erfindung betrifft ein Elektroden-Sensorsystem zur Messung von elektrischen Potentialen an der Hautoberfläche von Menschen und/oder Tieren, insbesondere zur Aufzeichnung eines Elektrokardiogramms (EKG), mit mehreren, wenigstens eine Elektrode aufweisenden, an der Hautoberfläche anbringbaren Elektroden-Einheiten, und mit einer am Körper lösbar befestigbaren Verteiler-Einheit mit einer Mehrzahl von Anschlußstellen, welche mit den Elektroden der Elektroden-Einheiten über Kabel verbindbar sind, wobei die zu den Anschlußstellen der Verteiler-Einheit führenden Kabel in einer Verteiler-Einrichtung zusammengeführt sind, welche mit einer Meßeinrichtung derart verbindbar ist, daß sämtliche Anschlußstellen der Verteiler-Einheit mit der Meßeinrichtung kommunizieren. Sie ist ferner auf eine Verteiler-Einheit eines solchen Elektroden-Sensorsystems gerichtet.

Elektroden-Sensorsysteme sind bekannt und finden insbesondere zur Aufzeichnung von Elektrokardiogrammen (EKG) im klinischen Bereich verbreitet Verwendung. Sie umfassen in der Regel eine Meßeinrichtung, welche eine Anzeigeeinrichtung umfaßt oder mit einer solchen verbunden ist, um die von den Elektroden erfaßten Potentiale visuell darstellen zu können. Die am Körper des Patienten in vorgegebenem Relativabstand angebrachten, z.B. aufgeklebten Elektroden werden dabei gewöhnlich direkt mit der stationären Meßeinrichtung verbunden. Dies ist in mehrfacher Hinsicht nachteilig.

Einerseits ist der Patient nach dem Anschließen der Elektroden an die Meßeinrichtung durch die Vielzahl der von den Elektroden abgehenden Kabel in seiner Bewegungsfreiheit stark eingeschränkt, was insbesondere die Aufnahme eines sogenannten Belastungselektrokardiogramms, bei welchem das elektrische Potential an der Hautoberfläche des Patienten unter körperlicher Anstrengung gemessen wird, stark erschwert. Andererseits läßt es sich praktisch nicht vermeiden, daß sich zumindest einige der von den Elektroden-Einheiten abgehenden Kabel während der Aufnahme des Elektrokardiogramms überkreuzen bzw. überschneiden, was in elektromagnetischen Wechselwirkungen und folglich in einem fehlerhaften Meßergebnis resultiert. Zudem bedeutet das Anschließen der Vielzahl an von den Elektroden ausgehenden Kabeln an die Meßeinrichtung einen erheblichen Zeitaufwand, wobei zusätzlich die Gefahr einer Vertauschung von Kabeln besteht, was zu einem gänzlich fehlerhaften Meßergebnis führt.

Neben Einzelelektroden sind Elektroden-Einheiten bekannt, welche jeweils eine oder mehrere, in vorgegebener Relativposition zueinander auf einem Trägermaterial angeordnete Elektroden umfassen. Der Vorteil des Einsatzes solcher Elektroden-Einheiten mit mehreren Elektroden besteht darin, daß in diesem Fall mehrere Elektroden gleichzeitig im jeweils gewünschten Abstand zueinander zeitsparend am Körper des Patienten angebracht werden können. Die Elektroden solcher Elektroden-Einheiten sind beispielsweise an einem Klebeband aus einem nicht stromleitenden Material, wie einer Kunststoffolie, angeordnet und über einen Mehrpol-Stecker gemeinsam an die Meßeinrichtung anschließbar. Eine solche Elektroden-Einheit ist beispielsweise der WO 02/09584 A1 und der US 5 868 671 A entnehmbar. Die Elektroden-Einheiten können einerseits wiederverwendbar, andererseits aus hygienischen Gründen insbesondere als Einweg-Komponente ausgebildet sein. Indes lassen sich die oben genannten Nachteile allein durch den Einsatz solcher mehrere Elektroden aufweisender Elektroden-Einheiten nicht zufriedenstellend lösen.

Darüber hinaus sind Elektroden-Sensorsysteme bekannt, welche eine Verteiler-Einheit besitzen, die einerseits mit den einzelnen Elektroden, andererseits mit der Meßeinrichtung verbunden ist (US 6 526 310 B1, US 5 813 979 A). Durch die Verteiler-Einheit ist die Bewegungsfreiheit des Patienten gegenüber einem direkten Anschluß der Elektroden an die Meßeinrichtung zwar erheblich verbessert, doch bieten die bekannten Systeme keinerlei Vertauschungssicherheit beim Anschließen einer Vielzahl von Elektroden und besteht zusätzlich die Gefahr einer Überkreuzung bzw. Überschneidung der Kabel während der Aufnahme des Elektrodiagramms, was - wie bereits erwähnt - zu Meßfehlern führt. Ferner sind die Elektroden als Mehrweg-Elektroden ausgebildet, was in vielen Fällen aus hygienischen Gründen unerwünscht ist. Schließlich ist die Verteiler-Einheit nicht zufriedenstellend am Körper des Patienten festlegbar, was insbesondere bei der Aufnahme eines Belastungselektrokardiogramms, bei dem sich der Patient bewegen muß, nachteilig ist.

Die als nächstliegender Stand der Technik betrachtete WO 01/013353 A1 beschreibt ein Elektroden-Sensorsystem gemäß dem Oberbegriff des Anspruchs 1, welches jedoch zur Aufzeichnung von Gehirnströmen ausgebildet ist. Die Verteiler-Einheit des bekannten Systems umfaßt zwei an einem gürtelartigen Träger festgelegte Schulterbänder, welche jeweils im Bereich des Kopfes des Patienten eine Vielzahl von Anschlußstellen zum Anschließen der Kabel der Elektroden aufweist, wobei die Vielzahl an Anschlußstellen über je ein Kabel mit einem Basismodul verbindbar ist, welches wiederum über ein weiteres Kabel an die Meßeinrichtung anschließbar ist. Der Anschluß der einzelnen Kabel an die Anschlußstellen der Verteiler-Einheit ist wiederum höchst zeitaufwendig, wobei zwangsläufig Überkreuzungen bzw. Überschneidungen einzelner Kabel auftreten. Überdies besteht die große Gefahr einer Vertauschung von Kabeln bzw. Anschlüssen.

Der Erfindung liegt die Aufgabe zugrunde, ein Elektroden-Sensorsystem der eingangs genannten Art auf einfache und kostengünstige Weise dahingehend weiterzubilden, daß die vorgenannten Nachteile vermieden werden.

Diese Aufgabe wird erfindungsgemäß bei einem Elektroden-Sensorsystem der eingangs genannten Art dadurch gelöst, daß die Verteiler-Einheit in Form eines Gürtels ausgebildet ist, welcher um den Körper des Menschen oder Tieres anordbar ist, und daß die Anschlußstellen in Erstreckungsrichtung des Gürtels hintereinander und mit Abstand voneinander angeordnet sind, wobei die Anschlußstellen der in Form eines Gürtels ausgebildeten Verteiler-Einheit von Mehrpol-Buchsen bzw. Mehrpol-Streckern gebildet sind, an welchen die Elektroden der mit mehreren Elektroden ausgestatteten Elektroden-Einheiten über hierzu komplementären Mehrpol-Stecker bzw. Mehrpol-Buchsen jeweils gemeinsam anschließbar sind.

Die erfindungsgemäß in Form eines Gürtels ausgebildete, am Körper des Patienten lösbar befestigbare Verteiler-Einheit sorgt einerseits für eine praktisch unbeeinträchtigte Bewegungsfreiheit des Patienten bei der Aufnahme eines Elektrokardiogramms, da die Kabel sämtlicher Elektroden-Einheiten an den Gürtel angeschlossen sind und der Patient somit nur über die Verteiler-Einrichtung desselben mit der Meßeinrichtung verbunden ist, was beispielsweise mittels eines einzigen Kabels oder auch drahtlos geschehen kann. Dies vereinfacht insbesondere die Aufnahme eines Belastungs-EKG. Der Gürtel wird dabei um den Körper des Patienten herumgelegt und endseitig miteinander verbunden.

Andererseits werden insbesondere Überkreuzungen bzw. Überschneidungen der von den Elektroden-Einheiten in die Verteiler-Einheit abgehenden Kabel und somit Meßfehler infolge elektromagnetischer Wechselwirkungen zuverlässig verhindert, da die Anschlußstellen der Verteiler-Einheit in Erstreckungsrichtung des Gürtels hintereinander und mit Abstand voneinander angeordnet sind. Da der Patient die als Gürtel ausgebildete Verteiler-Einheit am Körper trägt, sind die Elektroden-Einheiten bezüglich der Verteiler-Einheit auch im Falle von Bewegungen des Patienten in konstanter Relativposition zueinander angeordnet sind. Der Abstand der Anschlußstellen des Gürtels läßt sich dabei so wählen, daß die Kabel einer jeden am Oberkörper des Patienten festgelegten Elektroden-Einheit im wesentlichen senkrecht und parallel zueinander nach unten geführt und in die jeweiligen Anschlußstellen des Gürtels eingesteckt werden können. Der Abstand entspricht folglich etwa dem vorgesehenen seitlichen Abstand der an dem Körper des Patienten anzubringenden Elektroden-Einheiten. Auf diese Weise verlaufen die von den Elektroden-Einheiten in die Verteiler-Einheit mündenden Kabel etwa parallel, jedenfalls nicht über Kreuz, um elektromagnetische Einkopplungen sicher zu vermeiden. Ferner ist jeder Anschlußstelle des Gürtels eine jeweilige, etwa oberhalb derselben am Körper des Patienten angebrachte Elektroden-Einheit zugeordnet, so daß eine eindeutige Zuordnung der Anschlußstellen für die jeweiligen Elektroden-Einheiten gegeben ist und es nicht zu einer versehentlichen Vertauschung der Anschlüsse kommt.

Da die Elektroden der Elektroden-Einheiten über die Verteiler-Einrichtung der Verteiler-Einheit gemeinsam mit der Meßeinrichtung verbindbar sind, ist überdies eine zeitsparende und einfach handhabbare Aufnahme eines EKG möglich. Die mit Abstand in Erstreckungsrichtung des Gürtels hintereinander angeordneten Anschlußstellen gewährleisten, wie bereits erwähnt, ferner einen schnellen und vertauschungssicheren Anschluß der Kabel der Elektroden-Einheiten, indem jedem Anschluß des Gürtels die oberhalb desselben am Körper des Patienten angebrachte Elektroden-Einheit zugeordnet ist. Der schnelle und vertauschungssichere Anschluß einer Vielzahl von Elektroden ist zusätzlich dadurch sichergestellt, indem die Anschlußstellen der Verteilereinheit von Mehrpol-Buchsen bzw. Mehrpol-Steckern gebildet sind, an welchen die Elektroden von Elektroden-Einheiten mit mehreren Elektroden über hierzu komplementäre Mehrpol-Stecker bzw. Mehrpol-Buchsen gemeinsam anschließbar sind. Als Elektroden-Einheiten können hierbei insbesondere Elektroden-Einheiten der eingangs erwähnten Art eingesetzt werden, welche mit einer Mehrzahl an Elektroden ausgestattet sind, die beispielsweise an einem Klebeband in der gewünschten Anordnung zueinander festgelegt sind. Auf diese Weise ist der Patient einfach, schnell und zuverlässig an die Elektroden-Einheiten anschließbar, ohne in seiner Bewegungsfreiheit eingeschränkt zu sein, wobei sämtliche Elektroden über die Verteiler - Einrichtung des Gürtels gemeinsam anläßlich der Messung mit der Meßeinrichtung verbindbar sind.

Schließlich gibt die Erfindung die Möglichkeit einer sinnvollen Trennung von Ein- und Mehrwegkomponenten, indem sich die verhältnismäßig kostengünstigen Elektroden-Einheiten sowohl als Mehrweg- als auch insbesondere als Einweg-Komponenten ausbilden lassen, wie es aus hygienischen Gründen in vielen Fällen erwünscht ist, während alle übrigen Bauteile zweckmäßig als Mehrweg-Komponenten ausgebildet sind.

Eine bevorzugte Ausführung sieht vor, daß die Verteiler-Einrichtung von einem sämtliche Kabel der Anschlußstellen aufnehmenden Sammelkabel gebildet ist, über welches die Anschlußstellen der Verteiler-Einheit gemeinsam an die Meßeinrichtung anschließbar sind. In diesem Fall ist der Patient, wie bereits angedeutet, einzig über das Sammelkabel an die Meßeinrichtung angeschlossen.

Eine andere bevorzugte Ausführung sieht vor, daß die Verteiler-Einrichtung von einer Sendeeinrichtung gebildet ist, über welche die an den Anschlußstellen der Verteiler-Einheit empfangenen Signale gemeinsam an eine Empfangseinrichtung der Meßeinrichtung übermittelbar sind. In diesem Fall besteht keinerlei körperliche Verbindung des Patienten mit der Meßeinrichtung und genießt dieser folglich eine maximal mögliche Bewegungsfreiheit.

In vorteilhafter Ausgestaltung ist weiterhin vorgesehen, daß die zu den Anschlußstellen der Verteiler-Einheit führenden Kabel - innerhalb der als Gürtel ausgebildeten Verteiler-Einheit - in im wesentlichen paralleler Anordnung zu der Verteiler-Einrichtung geführt sind. Auf diese Weise ergibt sich aufgrund der im wesentlichen parallelen Anordnung der von der Verteiler-Einrichtung zu den Anschlußstellen zur Verbindung mit den Elektroden-Einheiten vorgesehenen Kabel der Verteiler-Einheit - im Falle einer Verteiler-Einrichtung in Form eines Sammelkabels insbesondere auch innerhalb desselben - eine geringstmögliche magnetische Wechselwirkung zwischen den einzelnen Kabeln unter Vermeidung etwaiger Überkreuz-Anordnungen einiger Kabel, was zu einem exakten und reproduzierbaren Meßergebnis ohne nennenswerte Meßfehler führt.

In bevorzugter Ausführung ist vorgesehen, daß die Verteiler-Einheit ferner wenigstens einen Anschluß für Referenz-Elektroden aufweist, wobei die zu den Referenz-Elektroden führenden Kabel insbesondere mit den zu den Anschlußstellen der Verteiler-Einheit führenden Kabeln im wesentlichen parallel angeordnet und gemeinsam mit diesen in der Verteiler-Einrichtung zusammengefaßt sind. Somit sind neben den Elektroden der Elektroden-Einheiten auch die Referenz-Elektroden, welche beispielsweise auf den Rücken des Patienten zur Ermittlung eines Bezugspunktes des gemessenen elektrischen Potentials aufgebracht werden, mittels der Verteiler-Einheit zusammengefaßt und sowohl die von den Elektroden der Elektroden-Einheiten als auch von den Referenz-Elektroden gemessenen Signale gemeinsam an die Meßeinrichtung übermittelbar.

Obgleich die erfindungsgemäße Ausgestaltung der Verteiler-Einheit an sich bereits einen zuverlässigen Schutz vor einem Anschluß einer Elektroden-Einheit in eine "falsche" Anschlußstelle des Gürtels bietet, können die Stecker und/oder die Buchsen mit einem geeigneten Verpolungsschutz ausgestattet sein, um ein fehlerhaftes Anschließen der Mehrpol-Stecker an die "falsche" oder auch an die "richtige" Mehrpol-Buchse, aber z.B. um 180° gedreht, zu verhindern. Der Verpolungsschutz kann beispielsweise durch entsprechende Geometrie der Stecker/Buchsen oder durch zueinander komplementären Führungsprofile gegeben sein, um den Anschluß eines Steckers an eine für diesen Stecker nicht vorgesehene Buchse und/oder den Anschluß eines Steckers an eine hierfür vorgesehene Buchse, aber in falscher Relativanordnung, zu vermeiden.

In bevorzugter Ausführung ist vorgesehen, daß die Anschlußstellen der Verteiler-Einheit im wesentlichen senkrecht zur Erstreckungsebene des Gürtels angeordnet sind, wobei sie insbesondere im Bereich der dem Oberkörper des Patienten zugewandten oberen Stirnseite des Gürtels angeordnet sein können.

In vielen Fällen ist es zweckmäßig, wenn die Verteiler-Einheit zwischen vier und zehn, insbesondere acht, Anschlußstellen aufweist. Entsprechend kann vorgesehen sein, daß die Mehrpol-Buchsen bzw. Mehrpol-Stecker der Anschlußstellen der Verteiler-Einheit zwischen vier und zehn, insbesondere acht, Pole zum Anschließen von Elektroden-Einheiten mit zwischen vier und zehn, insbesondere acht, Elektroden aufweisen. Auf diese Weise ist das Elektroden-Sensorsystem z.B. zur Aufnahme eines Vielkanal-Elektrokardiogramms mit acht Elektroden-Einheiten geeignet, welche jeweils mit acht Elektroden in vorgegebener Relativanordnung zueinander ausgestattet sind.

In Weiterbildung der Erfindung ist vorgesehen, daß die Verteiler-Einheit wenigstens eine elektrische und/oder elektronische Zusatzeinrichtung trägt.

Dabei kann die Zusatzeinrichtung insbesondere von einem Impedanzwandler und/oder einem Vorverstärker zur Verstärkung der von den Elektroden der Elektroden-Einheiten übermittelten Signale gebildet sein. Eine solche Vorverstärkung bzw. Impedanzwandlung hat den Vorteil, daß sie in der Nähe des Meßortes, d.h. in der Nähe der Elektroden der Elektroden-Einheiten erfolgt, wodurch die Störanfälligkeit der von den Elektroden übermittelten Signale bei der weiteren Übertragung an die Meßeinrichtung verbessert wird.

Alternativ oder zusätzlich kann die oder eine weitere Zusatzeinrichtung von einem Überspannungsschutz gebildet sein, welcher beispielsweise mit an sich bekannten Sicherungen ausgestattet sein kann. Ein solcher Überspannungsschutz dient einerseits zum Schutze des Patienten, andererseits zum Schutze des Meßsystems, wobei im Falle von erhöhten Spannungen, wie sie z.B. im Falle einer Defibrillation auftreten, bei welcher der Patient mittels gepulsten Stromstößen reanimiert wird, eine Beschädigung des Meßsystems zuverlässig verhindert wird.

Die als Gürtel ausgebildete, bandförmige Verteiler-Einheit ist weiterhin bevorzugt mittels eines Klettverschlusses am Körper befestigbar, um die Verteiler-Einheit möglichst einfach und zeitsparend am Körper des Patienten festzulegen. Selbstverständlich sind auch andere, einfach zu öffnende bzw. zu verschließende Verschlußmittel, wie Schnallen, Druckknöpfe, Klebebänder oder dergleichen, denkbar.

Während die Elektroden-Einheiten, wie bereits erwähnt, als Ein- oder Mehrwegkomponente ausgebildet sein können, ist die Verteiler-Einheit bzw. der Gürtel in zweckmäßiger Ausgestaltung wiederverwendbar.

Die Erfindung betrifft auch eine Verteiler-Einheit eines Elektroden-Sensorsystems zur Messung von elektrischen Potentialen an der Hautoberfläche von Menschen und/oder Tieren, insbesondere zur Aufzeichnung eines Elektrokardiogramms (EKG), mit mehreren, wenigstens eine Elektrode aufweisenden, an der Hautoberfläche anbringbaren Elektroden-Einheiten, wobei die Verteiler-Einheit am Körper lösbar befestigbar ist und eine Mehrzahl von Anschlußstellen aufweist, welche mit den Elektroden der Elektroden-Einheiten über Kabel verbindbar sind, wobei die zu den Anschlußstellen der Verteiler-Einheit führenden Kabel in einer Verteiler-Einrichtung zusammengeführt sind, welche mit einer Meßeinrichtung derart verbindbar ist, daß sämtliche Anschlußstellen der Verteiler-Einheit gemeinsam mit der Meßeinrichtung kommunizieren. Die erfindungsgemäße Verteiler-Einheit ist dadurch gekennzeichnet, daß die Verteiler-Einheit in Form eines Gürtels ausgebildet ist, welcher um den Körper des Menschen oder Tieres anordbar ist, und daß die Anschlußstellen in Erstreckungsrichtung des Gürtels hintereinander und mit Abstand voneinander angeordnet sind, wobei die Anschlußstellen der in Form eines Gürtels ausgebildeten Verteiler-Einheit von Mehrpol-Buchsen bzw. Mehrpol-Streckern gebildet sind, an welchen die Elektroden der mit mehreren Elektroden ausgestatteten Elektroden-Einheiten über hierzu komplementären Mehrpol-Stecker bzw. Mehrpol-Buchsen jeweils gemeinsam anschließbar sind.

Nachstehend ist die Erfindung anhand eines Ausführungsbeispiels unter Bezugnahme auf die Zeichnungen näher erläutert. Dabei zeigen:
- Fig. 1: eine Ausführungsform einer nach Art eines Gürtels ausgebildeten Verteiler-Einheit eines Elektroden-Sensorsystems und
- Fig. 2: eine an die Verteiler-Einheit gemäß Fig. 1 anschließbare Elektroden-Einheit des Elektroden-Sensorsystems.

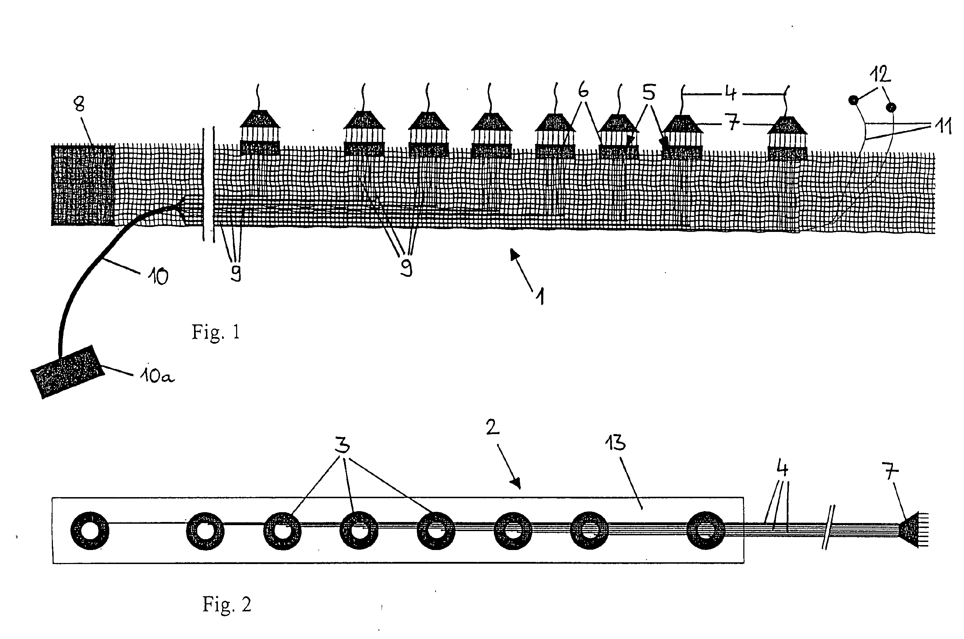

In Fig. 1 ist eine Verteiler-Einheit 1 eines Elektroden-Sensorsystems zur Messung von elektrischen Potentialen an der Hautoberfläche von Menschen und/oder Tieren, insbesondere zur Aufzeichnung eines Elektrokardiogramms, dargestellt. Das Elektroden-Sensorsystem umfaßt ferner eine Mehrzahl von in Fig. 2 wiedergegebenen Elektroden-Einheiten 2 mit mehreren Elektroden 3, welche über jeweils ein gemeinsam geführtes Kabel 4 an die Verteiler-Einheit 1 anschließbar sind. Das Elektroden-Sensorsystem umfaßt ferner eine Meßeinrichtung (nicht dargestellt) mit einer Anzeigeeinrichtung und gegebenenfalls einer Ausgabeeinrichtung, um die gemessenen elektrischen Potentiale sichtbar zu machen und gegebenenfalls auszudrucken.

Die in Fig. 1 wiedergegebene Verteiler-Einheit 1 umfaßt eine Mehrzahl von Anschlußstellen 5, welche beim vorliegenden Ausführungsbeispiel von jeweils einer Mehrpol-Buchse 6 gebildet sind, an welche jeweils ein hierzu komplementärer Mehrpol-Stecker 7 einer Elektroden-Einheit 2 gemäß Fig. 2 anschließbar ist. Selbstverständlich ist es ebenfalls denkbar, daß die Anschlußstellen 5 der Verteiler-Einheit 1 als Stecker ausgebildet sind, welche mit hierzu komplementären Buchsen der Elektroden-Einheiten 2 verbindbar sind (nicht dargestellt). Die Verteiler-Einheit 1 ist bandförmig nach Art eines Gürtels ausgebildet, wobei die Anschlußstellen 5 in Erstreckungsrichtung des Gürtels 1 hintereinander und mit Abstand voneinander sowie an der oberen Stirnseite des Gürtels 1 angeordnet sind. Der Abstand der Anschlußstellen 5 entspricht dabei dem medizinisch sinnvollen Abstand der Elektroden-Einheiten 2, welcher je nach Größe des Patienten z.B. zwischen etwa 3 cm und etwa 15 cm, vorzugsweise zwischen etwa 5 cm und etwa 10 cm, betragen kann, sofern acht Anschlußstellen 5 vorgesehen sind, wie es beim dargestellten Ausführungsbeispiel der Fall ist. Die Anschlußstellen 5 sind dabei insbesondere im wesentlichen gleichmäßig über die Erstreckungsrichtung des Gürtels 1 und folglich um den Körperumfang des Patienten verteilt. Am Ende des Gürtels 1 ist ein Klettverschluß 8 vorgesehen, um die Verteiler-Einheit 1 einfach und schnell am Körper eines Patienten lösbar zu befestigen, indem sie um dessen Hüfte herum gelegt und mittels des Klettverschlusses 8 endseitig aneinander befestigt wird.

Die zu den als Mehrpol-Buchsen 6 ausgebildeten Anschlußstellen 5 der Verteiler-Einheit 1 führenden Kabel 9 sind voneinander isoliert und im wesentlichen parallel angeordnet, wobei sie beispielsweise in den textilartigen Aufbau des Gürtels 1 integriert sind. Die Kabel 9 sämtlicher Anschlußstellen 5 sind zu einer Verteiler-Einrichtung der Verteiler-Einheit 1 zusammengeführt, welche beim vorliegenden Ausführungsbeispiel von einem die Kabel 9 aufnehmenden Sammelkabel 10 gebildet ist. In dem Sammelkabel 10 sind sämtliche Kabel 9 in im wesentlichen paralleler Anordnung, isoliert voneinander aufgenommen und über einen an dem der Verteiler-Einheit 1 abgewandten Ende des Sammelkabels 10 angeordneten Stecker 10a an die Meßeinrichtung (nicht gezeigt) anschließbar.

Das Sammelkabel 10 dient folglich zum gemeinsamen Anschließen sämtlicher Mehrpol-Buchsen 6 der Verteiler-Einheit 1 und somit sämtlicher hierin eingesteckten Mehrpol-Stecker 7 der Elektroden-Einheiten 2, gegebenenfalls unter Zwischenschaltung eines Verstärkers, an die Meßeinrichtung (nicht gezeigt). Anstelle des Sammelkabels 10 kann selbstverständlich auch eine andersartige Verteiler-Einrichtung, z.B. in Form eines mit einem Empfänger der Meßeinrichtung korrespondierenden Senders, vorgesehen sein (nicht dargestellt).

In dem Sammelkabel 10 sind ferner Kabel 11 aufgenommen, welche bezüglich der zu den Anschlußstellen 5 der Verteiler-Einheit 1 führenden Kabel 9 ebenfalls im wesentlichen parallel angeordnet sind und an deren Ende Elektrodenclips 12 festgelegt sind, welche als Referenz-Elektroden dienen und im Falle der Aufzeichnung eines Elektrokardiogramms beispielsweise am Rücken des Patienten angebracht werden können.

Die Verteiler-Einheit 1 kann ferner mit elektrischen und/oder elektronischen Zusatzeinrichtungen (nicht dargestellt), wie Vorverstärkern, Impedanzwandlern, Überspannungsschutzen oder dergleichen, ausgestattet sein.

Wie der Fig. 2 zu entnehmen ist, umfassen die Elektroden-Einheiten 2 des Elektroden-Sensorsystems eine Mehrzahl an Elektroden 3 - hier: acht Elektroden -, welche in einer vorgegebenen Anordnung relativ zueinander - im vorliegenden Fall etwa linear - auf einem Klebestreifen 13 aus einem elektrisch nicht leitenden Material, z.B. Kunststoffolie, angebracht sind. Die Elektroden 3 können aus einem beliebigen leitfähigen, für Elektroden geeigneten Material bestehen und z.B. als Silber/Silberchlorid-Elektroden in Verbindung mit einem Gel ausgestaltet sein.

Jede Elektrode 3 der Elektroden-Einheit 2 ist über je ein Kabel 4 mit einem Mehrpol-Stecker 7 verbunden, wobei die Kabel 4 im wesentlichen parallel angeordnet sind und der Mehrpol-Stecker 7, wie bereits erwähnt, bezüglich der Mehrpol-Buchse 6 der Verteiler-Einheit 1 gemäß Fig. 1 komplementär ausgestaltet ist, um ihn dort einzustecken. Die Stecker 7 und/oder die Buchsen 6 sind vorteilhafterweise mit einem Verpolungsschutz (nicht gezeigt) ausgestattet. Während die Verteilereinheit 1 zweckmäßig wiederverwendbar ist, ist die in Fig. 1 gezeigte Elektroden-Einheit 2 aus hygienischen Gründen insbesondere als Einweg-Komponente ausgebildet.

Die nach Art eines Gürtels ausgebildete und über den Klettverschluß 8 einfach und schnell um den Körper des Patienten umschnallbare Verteiler-Einheit 1 gewährleistet zum einen eine praktisch unbeeinträchtigte Bewegungsfreiheit des Patienten bei der Aufnahme eines Elektrokardiogramms, wobei die Kabel 4 sämtlicher Elektroden-Einheiten 2 über die Mehrpol-Stecker 7 an die Mehrpol-Buchsen 6 der Verteiler-Einheit 1 angeschlossen sind, so daß der Patient nur über das von der Verteiler-Einheit 1 zur Meßeinrichtung (nicht dargestellt) abgehende Sammelkabel 10 mit dieser verbunden ist. Die Aufnahme eines Belastungs-EKG ist somit einwandfrei möglich.

Zugleich ergibt sich aufgrund der etwa parallelen Anordnung der zu den Anschlußstellen 5 führenden Kabel 9 der Verteiler-Einheit 1 eine geringstmögliche elektromagnetische Wechselwirkung zwischen diesen Kabeln 9 unter Vermeidung einer unerwünschten Überkreuz-Anordnung der Kabel 9, welche zu einer elektromagnetischen Einkoppelung und somit zu Meßfehlern führen würde.

Darüber hinaus ergibt sich aufgrund des zwischen den Anschlußstellen 5 der Verteiler-Einheit 1 vorgesehenen Abstandes und der Anordnung der Anschlußstellen 5 um den Gürtel 1 herum eine im wesentlichen parallele Anordnung der von den Elektroden-Einheiten 2 zu den Mehrpol-Steckern 7 derselben abgehenden Kabel 4 nach Anschließen der Elektroden-Einheiten 2 an die Verteiler-Einheit 1, so daß auch hier Überkreuz-Anordnungen der Kabel 4 vermieden werden. Da die Kabel 9 sämtlicher Anschlußstellen 5 ferner in der Verteiler-Einrichtung 10 zusammengefaßt sind, ist die Verteiler-Einheit 1 einfach und schnell an die Meßeinrichtung (nicht gezeigt) anschließbar, so daß eine zeit- und kostensparende Aufnahme eines Elektrokardiogramms möglich ist.

## Patentansprüche

1. Elektroden-Sensorsystem zur Messung von elektrischen Potentialen an der Hautoberfläche von Menschen und/oder Tieren, insbesondere zur Aufzeichnung eines Elektrokardiogramms (EKG), mit mehreren, wenigstens eine Elektrode (3) aufweisenden, an der Hautoberfläche anbringbaren Elektroden-Einheiten (2), und mit einer am Körper lösbar befestigbaren Verteiler-Einheit (1) mit einer Mehrzahl von Anschlußstellen (5), welche mit den Elektroden (3) der Elektroden-Einheiten (2) über Kabel (4) verbindbar sind, wobei die zu den Anschlußstellen (5) der Verteiler-Einheit (1) führenden Kabel (9) in einer Verteiler-Einrichtung zusammengeführt sind, welche mit einer Meßeinrichtung derart verbindbar ist, daß sämtliche Anschlußstellen (5) der Verteiler-Einheit (1) mit der Meßeinrichtung kommunizieren, **dadurch gekennzeichnet, daß** die Verteiler-Einheit (1) in Form eines Gürtels ausgebildet ist, welcher um den Körper des Menschen oder Tieres anordbar ist, und daß die Anschlußstellen (5) in Erstreckungsrichtung des Gürtels (1) hintereinander und mit Abstand voneinander angeordnet sind, wobei die Anschlußstellen (5) der in Form eines Gürtels ausgebildeten Verteiler-Einheit (1) von Mehrpol-Buchsen (6) bzw. Mehrpol-Streckern gebildet sind, an welchen die Elektroden (3) der mit mehreren Elektroden (3) ausgestatteten Elektroden-Einheiten (2) über hierzu komplementären Mehrpol-Stecker (7) bzw. Mehrpol-Buchsen jeweils gemeinsam anschließbar sind.

2. Elektroden-Sensorsystem nach Anspruch 1, **dadurch gekennzeichnet, daß** die Verteiler-Einrichtung von einem sämtliche Kabel (9) der Anschlußstellen (5) aufnehmenden Sammelkabel (10) gebildet ist, über welches die Anschlußstellen (5) der Verteiler-Einheit (1) gemeinsam an die Meßeinrichtung anschließbar sind.

3. Elektroden-Sensorsystem nach Anspruch 1, **dadurch gekennzeichnet, daß** die Verteiler-Einrichtung von einer Sendeeinrichtung gebildet ist, über welche die an den Anschlußstellen (5) der Verteiler-Einheit (1) empfangenen Signale gemeinsam an eine Empfangseinrichtung der Meßeinrichtung übermittelbar sind.

4. Elektroden-Sensorsystem nach einem der Ansprüche 1 bis 3, **dadurch gekennzeichnet, daß** die zu den Anschlußstellen (5) der Verteiler-Einheit (1) führenden Kabel (9) in im wesentlichen paralleler Anordnung zu der Verteiler-Einrichtung geführt sind.

5. Elektroden-Sensorsystem nach einem der Ansprüche 1 bis 4, **dadurch gekennzeichnet, daß** die Verteiler-Einheit (1) ferner wenigstens einen Anschluß für Referenz-Elektroden (12) aufweist.

6. Elektroden-Sensorsystem nach Anspruch 5, **dadurch gekennzeichnet, daß** die zu den Referenz-Elektroden (12) führenden Kabel (11) mit den zu den Anschlußstellen (5) der Verteiler-Einheit (1) führenden Kabeln (9) im wesentlichen parallel angeordnet und gemeinsam mit diesen in der Verteiler-Einrichtung zusammengeführt sind.

7. Elektroden-Sensorsystem nach einem der Ansprüche 1 bis 6, **dadurch gekennzeichnet, daß** die Stecker (7) und/oder die Buchsen (6) mit einem Verpolungsschutz ausgestattet sind.

8. Elektroden-Sensorsystem nach einem der Ansprüche 1 bis 7, **dadurch gekennzeichnet, daß** die Anschlußstellen (5) der Verteiler-Einheit (1) im wesentlichen senkrecht zur Erstreckungsebene des Gürtels (1) angeordnet sind.

9. Elektroden-Sensorsystem nach einem der Ansprüche 1 bis 8, **dadurch gekennzeichnet, daß** die Verteiler-Einheit (1) zwischen vier und zehn, insbesondere acht, Anschlußstellen (5) aufweist.

10. Elektroden-Sensorsystem nach einem der Ansprüche 1 bis 9, **dadurch gekennzeichnet, daß** die Mehrpol-Buchsen (6) bzw. Mehrpol-Stecker der Anschlußstellen (5) der Verteiler-Einheit (1) zwischen vier und zehn, insbesondere acht, Pole zum Anschließen von Elektroden-Einheiten (2) mit zwischen vier und zehn, insbesondere acht, Elektroden (3) aufweisen.

11. Elektroden-Sensorsystem nach einem der Ansprüche 1 bis 10, **dadurch gekennzeichnet, daß** die Verteiler-Einheit (1) wenigstens eine elektrische und/oder elektronische Zusatzeinrichtung trägt.

12. Elektroden-Sensorsystem nach Anspruch 11, **dadurch gekennzeichnet, daß** die Zusatzeinrichtung von einem Impedanzwandler und/oder einem Vorverstärker zur Verstärkung der von den Elektroden (3) der Elektroden-Einheiten (2) übermittelten Signale gebildet ist.

13. Elektroden-Sensorsystem nach Anspruch 11, **dadurch gekennzeichnet, daß** die Zusatzeinrichtung von einem Überspannungsschutz gebildet ist.

14. Elektroden-Sensorsystem nach einem der Ansprüche 1 bis 13, **dadurch gekennzeichnet, daß** der Gürtel (1) mittels eines Klettverschlusses (8) am Körper befestigbar ist.

15. Elektroden-Sensorsystem nach einem der Ansprüche 1 bis 16, **dadurch gekennzeichnet, daß** der Gürtel (1) mit den Anschlußstellen (5) und der Verteiler-Einrichtung wiederverwendbar ist.

16. Verteiler-Einheit (1) eines Elektroden-Sensorsystems zur Messung von elektrischen Potentialen an der Hautoberfläche von Menschen und/oder Tieren, insbesondere zur Aufzeichnung eines Elektrokardiogramms (EKG), mit mehreren, wenigstens eine Elektrode (3) aufweisenden, an der Hautoberfläche anbringbaren Elektroden-Einheiten (2), wobei die Verteiler-Einheit (1) am Körper lösbar befestigbar ist und eine Mehrzahl von Anschlußstellen (5) aufweist, welche mit den Elektroden (3) der Elektroden-Einheiten (2) über Kabel (4) verbindbar sind, wobei die zu den Anschlußstellen (5) der Verteiler-Einheit (1) führenden Kabel (9) in einer Verteiler-Einrichtung zusammengeführt sind, welche mit einer Meßeinrichtung derart verbindbar ist, daß sämtliche Anschlußstellen (5) der Verteiler-Einheit (1) gemeinsam mit der Meßeinrichtung kommunizieren, **dadurch gekennzeichnet, daß** die Verteiler-Einheit (1) in Form eines Gürtels ausgebildet ist, welcher um den Körper des Menschen oder Tieres anordbar ist, und daß die Anschlußstellen (5) in Erstreckungsrichtung des Gürtels (1) hintereinander und mit Abstand voneinander angeordnet sind, wobei die Anschlußstellen (5) der in Form eines Gürtels ausgebildeten Verteiler-Einheit (1) von Mehrpol-Buchsen (6) bzw. Mehrpol-Streckern gebildet sind, an welchen die Elektroden (3) der mit mehreren Elektroden (3) ausgestatteten Elektroden-Einheiten (2) über hierzu komplementären Mehrpol-Stecker (7) bzw. Mehrpol-Buchsen jeweils gemeinsam anschließbar sind.

17. Verteiler-Einheit nach Anspruch 16, **gekennzeichnet durch** die Merkmale wenigstens eines der Ansprüche 2 bis 15.
